# FASCICULE DE BREVET EUROPEEN

(11) **EP 2 254 988 B1**
(45) Date de publication et mention de la délivrance du brevet: **01.02.2017**
(21) Numéro de dépôt: 09710506.8
(22) Date de dépôt: 05.02.2009
(51) Int. Cl.: C12N 1/38, C12N 1/00, C12P 1/00, C12Q 3/00

(54) **PROCEDE UTILISE POUR AUGMENTER LA BIOMASSE ET L'ACTIVITE METABOLIQUE DE MICROORGANISMES PAR LA REGULATION COMBINEE DU POTENTIEL D'OXYDO-REDUCTION ET DE L'OXYGENE DISSOUS DURANT LE PROCESSUS DE FERMENTATION**
VERFAHREN ZUR ERHÖHUNG DER BIOMASSE UND DER STOFFWECHSELAKTIVITÄT VON MIKROORGANISMEN DURCH KOMBINIERTE EINSTELLUNG DES SAUERSTOFFREDUKTIONSPOTENTIALS UND DES WÄHREND DER FERMENTIERUNG GELÖSTEN SAUERSTOFFS
METHOD FOR INCREASING THE BIOMASS AND THE METABOLIC ACTIVITY OF MICROORGANISMS BY THE COMBINED ADJUSTMENT OF THE OXIDATION-REDUCTION POTENTIAL AND OF THE OXYGEN DISSOLVED DURING THE FERMENTATION PROCESS

(30) Priorité: 15.02.2008 FR 0850976
(43) Date de publication de la demande: 01.12.2010
(73) Titulaire: L'Air Liquide Société Anonyme pour l'Etude et l'Exploitation des Procédés Georges Claude, 75007 Paris (FR)
(72) Inventeur: MARECAT, Armelle, 91300 Massy (FR); IBARRA, Dominique, 91190 Gif-sur-Yvette (FR); AUZANNEAU, Isabelle, 86220 Dangé Saint Romain (FR); MOURET, Jean-Roch, 86220 Dangé Saint Romain (FR); OBERT, Jean-Philippe, 86220 Dangé Saint Romain (FR); DAMONGEOT, Loïc, 94460 Valenton (FR); ROY, Olivier, 18000 Bourges (FR)
(74) Mandataire: Mellul-Bendelac, Sylvie Lisette
(86) Numéro de dépôt international: PCT/FR2009/050174
(87) Numéro de publication internationale: WO 2009/101328

(56) Documents cités:
- WO-A1-2007/036653
- FR-A- 2 891 634
- DU PREEZ J C ET AL: "The relation between redox potential and .OD-xylose fermentation by Candida shehatae and Pichia stipitis." BIOTECHNOLOGY LETTERS, vol. 10, no. 12, 1988, pages 901-906, XP002495139
- KWONG S C W ET AL: "Effect of Reducing Agents in an Aerobic Amino Acid-Fermentation" BIOTECHNOLOGY AND BIOENGINEERING, WILEY & SONS, HOBOKEN, NJ, US, vol. 40, no. 7, 1 janvier 1992 (1992-01-01), pages 851-857, XP002995801 ISSN: 0006-3592
- SUKHAREVICH M E ET AL: "Influence of aeration and redox potential on biosynthesis of antifungal antibiotic imbricin" ANTIBIOTIKI I HIMIOTERAPIA - ANTIBIOTICS AND CHEMOTHERAPY, MEDICINA, MOSCOW, RU, vol. 43, no. 12, 1 janvier 1998 (1998-01-01), pages 12-15, XP008095686 ISSN: 0235-2990
- GILL RYAN T ET AL: "Generating controlled reducing environments in aerobic recombinant Escherichia coli fermentations: Effects on cell growth, oxygen uptake, heat shock protein expression, and in vivo CAT activity" BIOTECHNOLOGY AND BIOENGINEERING, vol. 59, no. 2, 20 juillet 1998 (1998-07-20), pages 248-259, XP002495138 ISSN: 0006-3592
- MEMMERT K ET AL: "Continuous production of Bacillus exoenzymes through redox-regulation." ANNALS OF THE NEW YORK ACADEMY OF SCIENCES, vol. 506, 1987, pages 631-636, XP002495140 ISSN: 0077-8923
- THIRD K A ET AL: "Control of the redox potential by oxygen limitation improves bacterial leaching of chalcopyrite" BIOTECHNOLOGY AND BIOENGINEERING, vol. 78, no. 4, 20 mai 2002 (2002-05-20), pages 433-441, XP002495141 ISSN: 0006-3592
- NA ET AL: "Effect of electrochemical redox reaction on growth and metabolism of Saccharomyces cerevisiae as an environmental factor." JOURNAL OF MICROBIOLOGY AND BIOTECHNOLOGY, vol. 17, no. 3, mars 2007 (2007-03), pages 445-453, XP002495142 ISSN: 1017-7825
- RAO GOVIND ET AL: "MANIPULATION OF END-PRODUCT DISTRIBUTION IN STRICT ANAEROBES." ANNALS OF THE NEW YORK ACADEMY OF SCIENCES, vol. 506, 1987, pages 76-83, XP002495143
- DAHOD S K: "REDOX POTENTIAL AS A BETTER SUBSTITUTE FOR DISSOLVED OXYGEN IN FERMENTATION PROCESS CONTROL" BIOTECHNOLOGY AND BIOENGINEERING, vol. 24, no. 9, 1982, pages 2123-2125, XP002495144 ISSN: 0006-3592

## Description

La présente invention concerne un procédé de culture de microorganismes pour augmenter la biomasse et/ou l'activité métabolique de microorganismes (i.e augmenter la production de certains métabolites, c'est-à-dire de certaines molécules produites par les micro-organismes), ceci par la régulation combinée du potentiel d'oxydo-réduction et de l'oxygène dissous.

A titre illustratif, l'invention peut concerner par exemple des bactéries lactiques, mais également des microorganismes ayant préférentiellement un métabolisme anaérobie tel que des levures.

L'invention vise également le domaine de certains produits finis dont le procédé de production met en oeuvre une étape de fermentation, tels le vin ou encore la bière.

L'étape de culture proprement dite a lieu en cuve, sous agitation ou non, dans un milieu de culture dont la composition est adaptée aux besoins spécifiques de chaque micro-organisme. La composition du milieu de culture peut être extrêmement variée, mais on cite couramment la présence d'un ou plusieurs éléments parmi les polysaccharides, le glycérol, le lait, le glucose etc...

De même les paramètres tels que par exemple le pH, la température, la pression d'oxygène dissous peuvent être régulés.

Il existe différents types de conduite de culture :
- la culture discontinue ou « batch », utilisée notamment pour la production de ferments lactiques ou de levure de panification,
- la culture semi-continue ou « fed-batch », utilisée par exemple pour la production de ferments sensibles à un produit de la fermentation ou pour la production de biomasse sensible à l'inhibition par le substrat de la fermentation,
- la culture continue sans ou avec recyclage, cette dernière étant utilisée notamment pour la production de ferments, de molécules d'intérêt, et pour l'épuration biologique d'eaux résiduaires.

L'étape de conservation de la biomasse peut se faire sous forme liquide, par congélation, par cryo-conservation, par lyophilisation ou par séchage. Des agents protecteurs sont utilisés pour préserver les micro-organismes des effets néfastes des traitements de conservation.

La lyophilisation est on le sait une opération de déshydratation à basse température qui consiste à éliminer par sublimation, la majeure partie de l'eau contenue dans le produit après congélation.

Comme on l'a dit, les micro-organismes sont largement utilisés dans le domaine alimentaire, notamment les bactéries lactiques, comme ferments ou cultures probiotiques. La viabilité et l'activité métabolique de ces bactéries lactiques, produites à grande échelle pour leurs critères d'aptitude technologique, peuvent être affectées par les différentes étapes qu'elles subissent lors de leur production (inoculation, culture et concentration) et de leur conservation (congélation ou lyophilisation).

Les microorganismes peuvent également être mis à contribution pour la production de biocarburants, notamment par transformation du sucre en éthanol. Les microorganismes permettent également la production de molécules d'intérêt, notamment dans le domaine pharmaceutique.

L'optimisation de la biomasse et/ou de l'activité métabolique de ces cultures est donc très importante et a nécessairement une importance à la fois technologique et économique.

Les paramètres environnementaux ont un rôle clé sur la croissance des microorganismes, sur les réactions métaboliques et sur les mécanismes physiologiques responsables de l'activité des micro-organismes.

Tout comme le pH, la température ou la composition du milieu, la valeur du potentiel d'oxydo-réduction semble avoir un effet sur la croissance et la viabilité des souches bactériennes. En effet, plusieurs travaux sur les bactéries lactiques ont mis en évidence un effet du potentiel d'oxydo-réduction sur les flux métaboliques, la survie des ferments probiotiques, la production et/ou stabilité de molécules d'intérêts. On pourra à cet égard se reporter aux documents suivants : les documents EP-1 856 241 et EP-1 649 755 ainsi que WO 2007/036653 A1 au nom de la Demanderesse, ou encore le brevet US-7 078 201.

On notera que le document WO2007/036653 s'intéresse à la régulation du potentiel redox à l'une ou plusieurs étapes d'un procédé de fabrication d'un produit alimentaire ou biotechnologique comprenant une étape de fermentation, de façon à réaliser au moins une des étapes du procédé en condition réductrice et au moins une des étapes du procédé en condition oxydante, et notamment permettant d'alterner des phases de la fermentation considérée en conditions réductrices avec des phases de la fermentation considérée en conditions oxydantes.

Différents composés peuvent être utilisés comme agent réducteur et induire une baisse et/ou un maintien du potentiel d'oxydo-réduction. Parmi ceux-ci, nous pouvons citer les sulfites, l'ammoniaque mais également des gaz réducteurs comme l'hydrogène ou des mélanges comportant de l'hydrogène.

Ces travaux de l'art antérieur ont permis de démontrer que l'utilisation de milieux réduits, milieux où le potentiel d'oxydo-réduction est bas, permet d'augmenter la biomasse produite mais également la viabilité du produit au cours de sa conservation ultérieure, elle permet également d'augmenter la production de métabolites.

Un autre moyen répertorié par l'art antérieur pour augmenter la biomasse produite est l'utilisation d'une faible quantité d'oxygène. Bien que l'oxygène ait un effet inhibiteur sur les bactéries lactiques, bactéries anaérobies facultatives, il est décrit dans la littérature comme pouvant être bénéfique à leur croissance. Ainsi des travaux ont montré que pour une souche de *Lactococcus lactis* MG1363, l'ajout d'oxygène permet d'augmenter la biomasse produite. Ainsi, la concentration en biomasse passe de 0.54 g/l (poids sec) en culture anaérobie à 0.68g/l pour une culture à laquelle on ajoute un pourcentage d'oxygène dissous de 5%. (on se reportera aux travaux de Jensen et al intitulés « Metabolic behavior of Lactococcus lactis MG1363 in microaerobic continuous cultivation at a low dilution rate » parus dans AEM - 2001, Vol.67, N°6).

Il apparaît alors que les deux solutions préconisées par l'art antérieur, à savoir la mise en place d'un faible potentiel redox dans le premier cas, et l'action sur la quantité d'oxygène dissous dans le second cas, paraissent clairement incompatibles. En effet, l'oxygène étant un puissant agent oxydant, son ajout dans un milieu de culture va augmenter significativement le potentiel redox.

Comme on le verra ci-dessous plus en détails, c'est donc le mérite de la présente invention d'avoir démontré qu'il est possible d'apporter et de réguler une quantité d'oxygène dissous tout en régulant le potentiel redox à une valeur basse souhaitée et fixée, ceci entraînant de façon extrêmement avantageuse une augmentation significative de la productivité des souches cultivées, alors que l'action sur les deux paramètres précités pouvaient apparaître à l'Homme du Métier au premier abord au vu de l'art antérieur évoqué plus haut, et fort logiquement, comme s'annihilant.

La présente invention propose donc un procédé mettant en oeuvre des régulations simultanées, utilisant préférentiellement des gaz ou mélanges gazeux pour assurer ces régulations :
- avantageusement la régulation du potentiel redox utilisera l'injection d'un gaz ou mélange gazeux réducteur (par exemple un mélange gazeux comportant de l'hydrogène) dans le milieu de culture. On conçoit que l'ajout de produits chimiques tels l'acide ascorbique (vitamine C) par exemple pourrait également être mis en oeuvre pour réaliser cette régulation du redox, mais on préférera comme on l'a dit utiliser l'injection de gaz appropriés.
- tandis qu'avantageusement la régulation de la valeur d'oxygène dissous se fera par injection d'air ou d'oxygène pur ou encore d'air enrichi en oxygène dans le milieu de culture ou encore de tout .gaz susceptible de libérer de l'oxygène, par exemple des mélanges comportant de l'oxygène et du CO₂ etc....
- ces régulations sont effectuées durant tout ou partie de la fermentation, c'est-à-dire durant tout ou partie de la culture de la souche bactérienne.

On notera que le document US-7 078 201 cité ci-dessus indique qu'une valeur optimale du potentiel redox peut augmenter la production d'éthanol, diminuer la formation de glycérol et diminuer la durée de la fermentation par rapport à une fermentation classique. Le procédé proposé par ce document propose pour cela le maintien d'une valeur de potentiel redox dans le fermenteur comprise entre -250 et + 50 mV grâce à une aération en continu du milieu via une injection d'air. L'objectif recherché par les auteurs de ce document est d'aller à l'encontre d'une baisse du potentiel redox, et pour cela, selon un des modes recommandés par le document, l'auteur, en lieu et place de l'utilisation traditionnelle d'ammoniac (considéré comme trop réducteur) pour réaliser la régulation du pH lors de la croissance, recommande l'ajout de soude afin de limiter la baisse de la valeur du potentiel redox.

On comprend donc que les auteurs n'évoquent à aucun moment le fait de réaliser une régulation stable et continue du redox à une valeur fixée (régulation fine) et encore moins le fait de réguler une quantité d'oxygène dissous tout en régulant le potentiel redox à une valeur basse souhaitée et fixée (teneur en oxygène dissous qui naturellement aurait tendance à faire remonter le potentiel redox).

La présente invention concerne alors un procédé de culture de microorganismes, procédé du type où l'on procède notamment à une étape d'ensemencement d'un milieu de culture avec une ou plusieurs souches de microorganismes, et à une étape de culture du milieu ainsi ensemencé, procédé conforme à l'une ou chacune des revendications ci-après.

On doit comprendre à la lecture de ce qui précède que selon l'invention, on régule la valeur du potentiel redox Eh du milieu à une valeur de consigne qui est inférieure à la valeur qui serait naturellement atteinte par régulation du seul oxygène dissous.

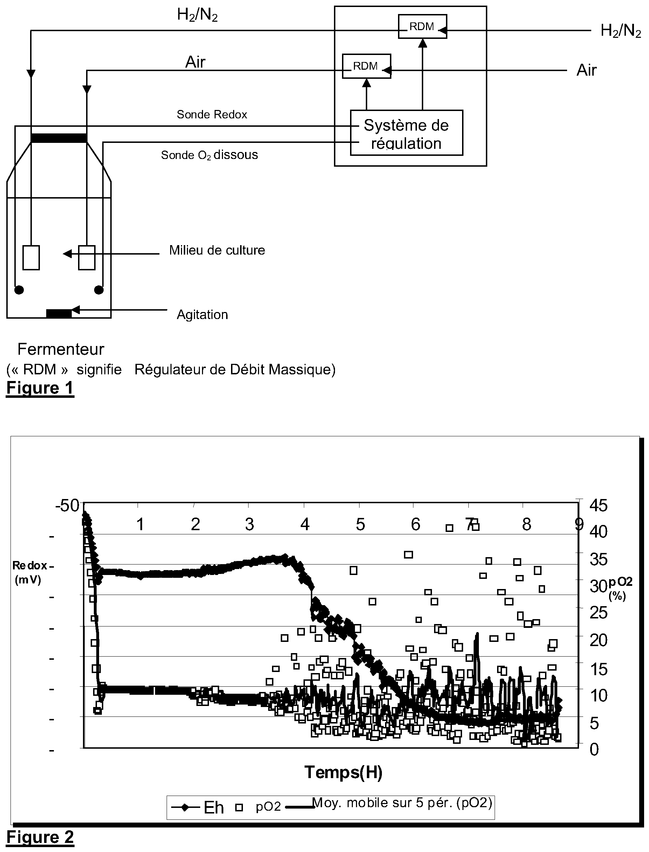

L'invention sera mieux comprise à la lecture de l'exemple qui suit et se réfère à la figure 1 qui illustre de façon schématique le montage expérimental utilisé pour effectuer les essais réalisés.

Les essais ont été réalisés sur une souche mésophile, *Lactococcus lactis,* en fermenteur de capacité de 1,5 litres.

Les valeurs cibles fixées (consignes) pour le potentiel d'oxydoréduction et l'oxygène dissous étaient respectivement de -200 mV et 10%.

Voici ce que l'on doit entendre par cette valeur d'oxygène dissous à 10% : L'appareillage utilisé est capable de mesurer la concentration en oxygène du milieu de culture grâce à une sonde. Cette sonde est étalonnée de la manière suivante : on injecte un mélange H2-N2 (4/96) dans le milieu de culture pour retirer tout l'oxygène présent. A ce stade, la sonde doit alors indiquer une concentration de 0% d'oxygène dissous. En un second temps, on remplace le mélange H2-N2 précité par de l'air, qui est injecté en grande quantité (typiquement au débit maximum que le système peut fournir). On attend que la valeur mesurée soit stable, le système doit alors indiquer une valeur de 100% d'oxygène dissous. Si ce n'est pas le cas, on réalise une calibration de la sonde qui consiste à faire prendre la valeur de 100% à la sonde. Ce « 100% d'oxygène dissous» correspond donc au maximum d'oxygène que le milieu considéré puisse dissoudre. Une fois cette calibration réalisée, on peut activer la régulation sur l'oxygène dissous avec la consigne que l'on souhaite mettre en place dans le milieu de culture (ici par exemple 10 %). Les essais étaient donc réalisés à 10% du maximum que le milieu considéré puisse dissoudre.

De même on va détailler dans ce qui suit un mode de réalisation des deux régulations simultanées conformément à la présente invention.

On dispose d'un automate qui périodiquement (selon une période qui lui a été imposée, par exemple inférieure à la seconde), examine chacun des régulateurs de débit massique, à savoir sa valeur de consigne et la valeur de mesure du paramètre considéré et qui corrige en conséquence sa sortie i.e l'instruction qu'il donne en rétroaction. On utilise en l'occurrence une régulation de type PID qui permet ici de réguler à la fois le Redox et l'oxygène dissous. Comme on va l'expliquer plus en détail dans ce qui suit il faut en effet signaler que le potentiel redox et l'oxygène dissous n'ont pas le même comportement, ils n'ont pas les mêmes constantes de temps, ni une même amplitude de réaction pour une variation de débit identique. En conséquence, les paramètres de chaque régulateur ne sont pas identiques et le réglage fin mis en place sur chaque PID permet que les régulateurs n'oscillent pas, cette mise en oeuvre limite ainsi les interférences entre eux.

La mise en oeuvre décrite ci-dessus n'est qu'illustrative d'un mode de mise en oeuvre, elle n'exclue pas bien entendu, et ceci sans à aucun moment sortir du cadre de la présente invention, d'utiliser d'autres moyens de régulations.

On décrit ci-dessous les entrées / sorties de régulateur typiquement mises en place :

### Pour le Redox :

- En entrée :
   i) Consigne de Redox en millivolt donnée depuis l'Interface Homme Machine (IHM)
   j) Mesure de Redox en millivolt envoyée par la sonde plongée dans le milieu de culture
- En sortie :
   k) Débit d'H₂-N₂ envoyé dans le milieu

### Pour l'oxygène dissous :

- En entrée :
   i) Consigne d'O₂ dissous exprimée en pourcentage (0-100%) et qui a été entrée depuis l'Interface Homme Machine (IHM)
   j) Mesure d'O₂ dissous envoyée par la sonde correspondante en mV et convertie en pourcentage pour disposer de la même unité que celle de la consigne

- En sortie :
   k) Débit d'air envoyé dans le milieu

Plus précisément, pour l'exemple de mise en oeuvre indiqué ci-dessus :
- on a utilisé un régulateur de type PID qui est en fait un Régulateur Proportionnel Intégral. On pourrait également utiliser d'autres types de régulateurs comme par exemple à modèle interne ou à logique floue ;
- comme on l'a indiqué plus haut, le potentiel redox et l'oxygène dissous n'ont pas le même comportement, ils n'ont pas les mêmes constantes de temps, ni une même amplitude pour une variation de débit identique. En conséquence, les paramètres de chaque régulateur ne sont pas identiques et le réglage adapté mis en place sur chaque PID permet que les régulateurs n'oscillent pas, ce qui limite ainsi les interférences entre eux. Plus précisément, ce qui permet selon l'invention de différencier les paramètres de chaque régulateur c'est la quantification préalable de l'impact de l'air ou autre gaz susceptible de libérer de l'oxygène et du mélange comprenant de l'hydrogène sur la valeur d'oxygène dissous d'une part et sur le potentiel redox d'autre part. Cette quantification a été réalisée via la réalisation d'essais préalables où l'on a mis en place des variations de débits de gaz injecté dans le milieu. Ces essais ont permis de déterminer l'impact de l'injection d'air sur le potentiel redox et sur l'oxygène dissous, puis l'impact de l'injection d'un mélange comportant de l'hydrogène sur le potentiel redox et sur l'oxygène dissous. Ces essais permettent de connaître l'impact de chaque gaz sur sa grandeur mesurée de référence mais également son impact (ou la perturbation qu'il engendre) sur l'autre grandeur mesurée. Ces expérimentations ont permis de conclure que dans le cas étudié (mélanges gazeux, milieu traité...) :
- l'influence du mélange hydrogéné sur l'oxygène dissous est environ quatre fois plus faible que celle de l'air.
- pour ce qui est du potentiel Redox, l'air a un impact environ 10 fois plus faible que celui du mélange hydrogéné.

Prenant en compte cette identification des fonctions de transfert, il est ainsi possible de déterminer les paramètres de chaque correcteur, en se donnant pour objectif que le système réagisse correctement à une perturbation du système pour une consigne fixe. En effet, selon l'invention on met en place une consigne donnée durant tout ou partie de la croissance, en revanche il fallait inclure dans la détermination des paramètres du correcteur l'effet de la croissance des bactéries dans le milieu de culture. On sait en effet que la croissance des bactéries a une influence sur le potentiel redox : le potentiel redox diminue durant la croissance et la consommation d'oxygène augmente. Il s'agit d'un phénomène perturbant pour la régulation : le réglage des correcteurs est fait pour réagir au mieux à cette perturbation.

De cette manière, c'est la combinaison des identifications effectuées et de la manière dont est réglé le correcteur (réponse à une perturbation et non à un changement de consigne) qui permet d'obtenir les résultats observés avec l'utilisateur de régulateurs « simple » de type PID (monovariable).

L'objectif de la régulation durant les essais rapportés ci-dessous est de maintenir durant au moins une partie de la croissance de la souche une valeur constante de potentiel redox ainsi qu'une valeur constante d'oxygène dissous (on le visualise d'ailleurs bien sur la figure 2 qui sera commentée ci-après).

La biomasse, l'activité acidifiante et la productivité par opération ont été mesurées en fin de fermentation (fin de culture), mais également au niveau des pellets congelés ainsi qu'au niveau du produit lyophilisé. Ces résultats sont comparés à une culture témoin dans laquelle ni le potentiel redox ni l'oxygène dissous n'ont été régulés (culture anaérobie, réalisée avec un simple balayage d'azote de 0,5 l/min dans l'espace de tête du fermenteur).

Les résultats présentés en figure 2 montrent le suivi de la régulation des valeurs d'oxygène dissous (pO2) et de potentiel d'oxydo-réduction (Eh) pour une souche de *Lactococcus lactis.*

Les différents gains obtenus au cours des différentes étapes de production sont décrits dans le tableau ci-dessous.

On met donc en évidence le résultat primordial de cette invention selon lequel il est possible, par un système de régulation maîtrisé, de découpler les deux paramètres tout en les régulant simultanément : potentiel d'oxydoréduction et oxygène dissous.

On constate alors que les gains obtenus sont supérieurs à 50% à l'étape du produit lyophilisé ce qui est très satisfaisant. En régulant simultanément les deux paramètres, potentiel d'oxydo-réduction et oxygène dissous, on obtient des gains importants au niveau de la biomasse et de l'activité acidifiante.

**Tableau de résultats obtenus**

| | Fin de fermentation | Pellets congelés | Produit lyophilisé |
|---|---|---|---|
| Gain en biomasse (en %) | 28% | 45% | 55% |
| Gain en activité acidifiante (en %) | 17% | 19% | 71% |
| Gain en productivité (en %) | 17% | Non déterminé | 71% |

Comme il apparaîtra clairement à l'homme du métier, la valeur optimale en oxygène dissous et en potentiel redox sera à adapter en fonction des souches et de l'objectif visé (production de biomasse et/ou production de métabolites).

Il est également envisageable de réaliser des séquences différentes de régulation en fonction de la phase de croissance concernée, c'est à dire de mettre en place des couples (valeur du redox / pO₂) différents en fonction de la phase de croissance de la souche, à titre illustratif on donne ci-dessous un exemple numérique :
- en début de croissance une valeur de redox basse, voisine de -400 mV et une valeur d'oxygène dissous voisine de 10 %,
- puis en phase exponentielle une valeur de redox voisine de -400 mV et une valeur d'oxygène dissous voisine de 5%,
- et en phase stationnaire une valeur de redox voisine de -400 mV et une valeur d'oxygène voisine de 0%.

## Revendications

1. Procédé de culture de microorganismes, procédé du type où l'on procède notamment à une étape d'ensemencement d'un milieu de culture avec une ou plusieurs souches de microorganismes, et à une étape de culture du milieu ainsi ensemencé, **se caractérisant en ce que** l'on procède durant tout ou partie de la culture, aux deux régulations suivantes de façon simultanées :
- on régule la quantité d'oxygène dissous dans le milieu à une consigne d'oxygène dissous donnée ;
- on régule la valeur du potentiel redox Eh du milieu à une valeur de consigne Eh donnée ;
dans les conditions suivantes :
- le potentiel redox est régulé dans la gamme allant de - 400 à 0 mv ;
- valeur d'oxygène dissous est régulée dans la gamme 1 à 30 % ;
- la régulation du potentiel redox utilise l'injection d'un gaz ou mélange gazeux réducteur dans le milieu de culture, tel qu'un mélange gazeux comportant de l'hydrogène, tandis que la régulation de la valeur d'oxygène dissous utilise une injection d'air ou d'un gaz ou mélange gazeux susceptible de libérer de l'oxygène tel l'oxygène pur ou encore de l'air enrichi en oxygène dans le milieu de culture.

2. Procédé de culture selon la revendication 1, **caractérisé en ce que** l'on réalise des séquences différentes de régulation en fonction de la phase de croissance concernée, par la mise en oeuvre de couples (consigne de valeur du potentiel redox, consigne d'oxygène dissous) différents en fonction de la phase considérée de croissance de la dite souche.

3. Procédé de culture selon l'une des revendications 1 ou 2, **caractérisé en ce que** les deux régulations simultanées sont réalisées par une régulation de type PID selon la procédure suivante :
- on a réalisé au préalable l'évaluation de l'impact de l'injection d'air ou d'un mélange susceptible de libérer de l'oxygène, et d'un gaz ou mélange gazeux réducteur, sur la valeur d'oxygène dissous d'une part et sur le potentiel redox d'autre part, via la réalisation d'essais préalables mettant en oeuvre des débits de gaz variables injectés dans le milieu ;
- on dispose de deux régulateurs de débit aptes à réguler le débit du gaz susceptible de libérer de l'oxygène et le débit du gaz ou mélange gazeux réducteur dans le milieu de culture ;
- on dispose d'un système de régulation avec acquisition de données, par exemple un automate qui périodiquement examine chacun des régulateurs de débit, au niveau de sa valeur de consigne et de la valeur de mesure du paramètre qui lui est associé, et qui corrige en conséquence sa sortie ;
- on a déterminé à partir desdites évaluations d'impact les mesures de correction de chaque régulateur en fonction d'une perturbation du système pour des consignes données de potentiel redox et d'oxygène dissous.

## Patentansprüche

1. Verfahren zum Kultivieren von Mikroorganismen, Verfahren des Typs, bei dem insbesondere ein Schritt des Beimpfens eines Kulturmediums mit einem oder mehreren Mikroorganismenstämmen, und ein Schritt des Kultivierens des so beimpften Mediums vorgenommen werden, **dadurch gekennzeichnet, dass** während des gesamten oder eines Teils des Kultivierens die folgenden zwei Regelungen gleichzeitig vorgenommen werden:
- Regeln der im Medium gelösten Sauerstoffmenge auf einen gegebenen Sollwert an gelöstem Sauerstoff;
- Regeln des Redoxpotentialwertes Eh des Mediums auf einen gegebenen Eh-Sollwert;
unter den folgenden Bedingungen:
- das Redoxpotential wird im Bereich von -400 bis 0 mv geregelt;
- der Wert an gelöstem Sauerstoff wird im Bereich 1 bis 30 % geregelt;
- die Regelung des Redoxpotentials nutzt die Einspritzung eines reduzierenden Gases oder Gasgemisches in das Kulturmedium, wie etwa eines Sauerstoff enthaltenden Gasgemisches, während die Regelung des Wertes an gelöstem Sauerstoff eine Einspritzung von Luft in das Kulturmedium oder eines Gases oder Gasgemisches nutzt, welches in der Lage ist, Sauerstoff freizusetzen, wie reiner Sauerstoff oder auch mit Sauerstoff angereicherte Luft.

2. Kultivierungsverfahren nach Anspruch 1, **dadurch gekennzeichnet, dass** verschiedene Regelsequenzen abhängig von der betreffenden Wachstumsphase ausgeführt werden durch Anwenden von verschiedenen Paare (Sollwert des Redoxpotentials, Sollwert an gelöstem Sauerstoff) abhängig von der betrachteten Wachstumsphase des Stamms.

3. Kultivierungsverfahren nach einem der Ansprüche 1 oder 2, **dadurch gekennzeichnet, dass** die beiden gleichzeitigen Regelungen von einer Regelung des Typs PID nach dem folgenden Vorgehen ausgeführt werden:
- die Bewertung der Auswirkung des Einspritzens von Luft oder eines Gemisches, welches in der Lage ist, Sauerstoff freizusetzen, und eines reduzierenden Gases oder Gasgemisches auf den Wert an gelöstem Sauerstoff einerseits und auf das Redoxpotential andererseits, wurde zuvor ausgeführt durch Ausführung von Vorversuchen unter Anwendung von variablen Gasdurchflussmengen, die in das Medium eingespritzt werden;
- es wird über zwei Durchflussregler verfügt, welche dazu geeignet sind, die Durchflussmenge des Gases, welches in der Lage ist, Sauerstoff freizusetzen, und die Durchflussmenge des reduzierenden Gases oder Gasgemisches in dem Kulturmedium zu regeln;
- es wird über ein Regelsystem mit Datenerfassung verfügt, zum Beispiel einen Automaten, der regelmäßig jeden der Durchflussregler auf Höhe seines Sollwerts und des Messwerts des ihm zugeordneten Parameters überprüft und in der Folge seinen Ausgang korrigiert;
- die Maßnahmen zur Korrektur jedes Reglers wurden abhängig von einer Systemstörung für gegebene Sollwerte des Redoxpotentials und des gelösten Sauerstoffs anhand der Bewertungen der Auswirkung bestimmt.

## Claims

1. Method for cultivating microorganisms, method of the type wherein a step of seeding a culture medium with one or more microorganism strains is in particular carried out, and a step of cultivating the medium thus seeded, **characterised in that** during the entirety or a portion of the cultivation, the two following and simultaneous adjustments are carried out:
- adjusting the amount of oxygen dissolved in the medium to a given dissolved-oxygen setpoint;
- adjusting the value of the redox potential Eh of the medium to a given setpoint value Eh;
in the following conditions:
- the redox potential is adjusted in the range from - 400 to 0 mv;
- the value of oxygen dissolve is adjusted in the range 1 to 30%;
- the adjusting of the redox potential uses the injection of a reducing gas or of a gaseous mixture into the culture medium, such as a gaseous mixture comprising hydrogen, whereas the adjusting of the value of oxygen dissolved uses an injection of air or of a gas or gaseous mixture able to release oxygen such as pure oxygen or oxygen-enriched air in the culture medium.

2. Method for cultivating according to claim 1, **characterised in that** different sequences of adjusting are carried out according to the growth phase concerned, through the implementation of different pairs (redox potential setpoint value, dissolved-oxygen setpoint) according to the phase considered of growth of said strain.

3. Method for cultivating according to one of claims 1 or 2, **characterised in that** the two simultaneous adjustments are carried out by an adjusting of the PID type according to the following procedure:
- beforehand was carried out the assessment of the impact of the injection of air or of a mixture able to release oxygen, and of a reducing gas or gaseous mixture, on the dissolved oxygen value on the one hand and on the redox potential on the other hand, via the carrying out of prior tests implementing variable gas flow rates injected into the medium;
- two flow rate regulators are arranged able to adjust the flow rate of the gas able to release oxygen and the flow rate of the reducing gas or gaseous mixture in the culture medium;
- there is an adjusting system with data acquisition, for example an automaton that periodically examines each one of the flow rate regulators, on its setpoint value and the measurement value of the parameter that is associated with it, and which consequently corrects its output;
- using said impact assessments the corrective measures were determined for each regulator according to a disturbance of the system for given setpoints of redox potential and dissolved oxygen.
